(19) Europäisches Patentamt / European Patent Office / Office européen des brevets

(11) **EP 3 960 772 A1**

(12) **EUROPEAN PATENT APPLICATION**

(43) Date of publication:
**02.03.2022 Bulletin 2022/09**

(51) International Patent Classification (IPC):
***C08B 37/00*** (2006.01)    ***C08L 5/00*** (2006.01)

(21) Application number: **21193566.3**

(52) Cooperative Patent Classification (CPC):
**C08B 37/0057; C08B 37/0003; C08L 5/00**

(22) Date of filing: **27.08.2021**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.08.2020 PT 2020116669**

(71) Applicant: **Universidade do Minho
4704-553 Braga (PT)**

(72) Inventors:
• **ANTHONY COLLINS, JAMES
4715-315 BRAGA (PT)**
• **FARIA BARROCA, MÁRIO JORGE
3505-499 VISEU (PT)**

(74) Representative: **Patentree
Edificio Net
Rua de Salazares, 842
4149-002 Porto (PT)**

(54) **PROCESS FOR THE PRODUCTION OF XYLAN AND XYLAN PRODUCTS FROM ALGAE PRODUCTS AND THEIR USES**

(57)    The present disclosure refers to a process for preparing an oligosaccharide mixture which avoids the need for the currently used multi-step, expensive and hazardous processes and allows for the development of an environmentally friendly, sustainable, green chemistry production process. Particularly, the present disclosure refers to the production of xylo-oligosaccharides compositions, xylan and/or protein-rich preparations from algae, said process comprising algae washing, thermal drying and milling, aqueous extraction and an enzyme hydrolysis step. A second embodiment of the present invention refers to the use of said xylan products in several fields of industry, such as in food/feed, healthcare, research and development, materials, pharmaceutical, chemical, biofuels and/or cosmetic applications among others.

EP 3 960 772 A1

**Description**

**TECHNICAL FIELD**

The present disclosure relates to a process for the production of xylo-oligosaccharide compositions, soluble xylan and insoluble protein-rich preparations from water biomass. Specifically, the said process comprises biomass washing and thermal drying and milling, aqueous extraction of a soluble xylan fraction and insoluble protein-rich fraction, and an enzyme hydrolysis step. More specifically, enzyme hydrolysis is carried out at low to moderate temperatures to avoid heating needs and reduce the energy costs and environmental impact. A second embodiment of the present invention refers to the use of said compositions in several fields of industry.

**BACKGROUND**

Xylans are complex plant heteropolysaccharides composed of a linear backbone chain of β1,4 or/and β1,3-linked β-D-xylopyranosyl units which may or may not be substituted to varying degrees with various side chain groups (Collins et al, 2005). They constitute a major component of hemicellulose in plant biomass and have numerous potential applications in various areas, including in the food, feed, health, materials, biofuels, chemicals, cosmetics, and research and development (R&D) fields.

Xylan can be hydrolysed to low molecular weight oligosaccharides known as xylo-oligosaccharides or xylo-oligomers which themselves have enormous potential for application in a number of diverse areas. These are composed of a linear backbone chain of β1,4 or/and β1,3-linked β-D-xylopyranosyl units, with the former (i.e., β1,4 linkages) being the most commonly studied. From the content disclosed in the document WO2006/002495, a person skilled in the art understands that, while the average degree of polymerisation of xylo-oligosaccharides is most commonly accepted as being between 2 and 10 xylose units, average degrees of polymerisation of up to 50 have also been described in the state of the art. Both linear, non-branched xylo-oligomers, and side chain substituted, branched xylo-oligosaccharides are known. In the latter case, the backbone chain can be branched to varying degrees with, e.g., glucuronyl, methyl-D-glucuronyl, α-L-arabinosyl, galacturonyl, xylosyl, rhamnosyl, galactosyl, glucosyl, acetyl, feruloyl dehydrodiferuloyl, sinapyl and/or p-coumaroyl side-chain groups (Collins et al, 2005).

β1,4-linked xylo-oligosaccharides have already been shown to be effective, safe and stable prebiotics, being beneficial for gastrointestinal health, but are also non-cariogenic with potential as low-calorie sweeteners, as disclosed in the documents EP 0265970B1, Courtin et al, 2009, Kim et al, 2015 and Hsu et al, 2004. Thus, they can be used to improve the health benefits and/or organoleptic properties of foods/feeds and beverages or/and they can be used as/in functional food/feed ingredients, supplements and/or preparations.

A number of other potential applications in various other sectors have also been described for both β1,4 and β1,3 linked xylo-oligosaccharides. In health care they have been shown to have potential in diabetes treatment/prevention, and as anti-cancer, immunostimulatory, anti-inflammatory, anti-microbial, and/or anti-oxidant agents (Amorim et al, 2019; Maeda et al, 2012; Hsu et al, 2004). In cosmetics and personal care, they have been proposed for use in for example skin and hair regeneration and as moisturisers. In addition, their use in the production of platform chemicals, biofuels and materials is also recognised wherein their xylose monomers are used as feedstock.

Importantly, the xylo-oligosaccharide structure and composition (e.g., the degree of polymerisation, presence/absence of branching, type(s) of branching and/or type(s) of linkages) directly affects the properties and functionality of the xylo-oligosaccharides, with, for example, variations in the prebiotic effect, taste, sweetness and/or calorific value being reported, for example in the document WO2006/002495 and in Kabel et al, 2002.

Indeed, as compared to shorter chain xylo-oligosaccharides, higher degrees of polymerisation are associated with decreased sweetness, decreased calorific value, and prebiotic action in the distal part of the colon, (WO2006/002495). Likewise, side-chain substitutions are associated with reduced rates of fermentation by intestinal flora (Kabel et al, 2002). Thus, variations in other properties and functionalities due to variations in structure and composition are to be expected.

Xylo-oligosaccharides are mainly produced by hydrolysis of xylan, a complex heteropolysaccharide found in plants and constituting the major component of hemicellulose in plant biomass. Presently, xylo-oligosaccharides are mainly produced from xylan originating from land-based plants, mostly from lignocellulosic materials, via chemical and physical methods with use of hazardous, corrosive chemicals (e.g. acids, alkali, ammonia, alkaline peroxide, solvents) and/or processes (e.g. hydrothermal treatment, high temperatures, high pressures). Recently, novel technologies including microwave oven treatment (Teleman et 2000), ultrasonication (Wang et al, 2006), and use of ionic liquids (Ávila et al, 2020) have also been investigated, with varying results.

In general, xylo-oligosaccharide production and purification involves two major stages. First, major contaminants are removed and xylan extracted from the source plant by chemical and/or physical methods, with or without enzyme treatment. Then, the isolated xylan is hydrolysed to xylo-oligosaccharides by high temperature, acid, alkali and/or enzyme treatment. In fact, enzymatic hydrolysis, with endo-β-(1,4)-xylanases (also known as endoxylanases), EC 3.2.1.8, has recently gained much interest as it is the most specific approach for xylo-oligosaccharide production, avoiding by-product production.

Frequently, depending on the complexity of the source material, multiple different successive treatments are required for successful xylan and/or xylo-oligosaccharide production and purification, thereby increasing complexity and costs.

The state of the art comprises various different multi-step procedures for xylan and/or xylo-oligosaccharide production from numerous feedstocks, including: industry and agriculture wastes, hardwoods, softwoods, cereals, corncob, barley hulls and barley spent grains, brewers' spent grain, almond shells, kenaf stem, corn fibre, rice hulls, sugarcane bagasse, straw, etc. (see Aachary and Prapulla, 2011 and Amorim et al, 2019 for reviews; CN101531722B; EP3252083A; CN108251472A; EP0265970B1).

Particularly, some examples of the matter disclosed in the state of the art are now given:
The document CN 108 251472 mentions a method for xylo-oligosaccharide production from lignocellulosic materials, namely sugarcane bagasse, poplar, and eucalyptus. This method involves material crushing and drying, alkali-ethanol treatment at 100-120 °C, filtration, concentration, alcohol precipitation, subcritical water extraction at 120-150 °C, pH adjustment, and enzyme treatment at 45-50 °C, pH 5.4, for 45 to 50 hours. Such a multistep method presents various economic and environmental challenges, combining multiple chemical and physical methods with high energy inputs and chemicals use as well as requirements for specialised equipment and production of hazardous wastes, in addition to further heating requirements for the enzyme hydrolysis step.

Document EP 0 265 970 describes a method for xylo-oligosaccharide production from corncob, cottonseed hull and malt cake, involving NaClO treatment for 15 hours at room temperature, washing with water and drying, 24% KOH soaking for 17 hours at 37 °C, suction filtering, washing with water, dialysis, acetic acid neutralisation and ethanol drying, followed by endoxylanase treatment for 4 hours at 65-70 °C and pH 5.3. Said document also describes an alternative method for producing xylo-oligosaccharides from malt cake involving use of high temperature steam blasting (180 °C at 10 kg/cm$^2$G pressure and blowing to atmospheric pressure) or high temperature incubation (180 °C for 20 minutes), followed by centrifugation and endoxylanase treatment at 55 °C. Both processes described in EP 0 265 970 are characterised by multiple steps in addition to the pre-treatment processes required for preparation of the biomass feedstocks. They involve use of harsh chemical or physical processes and produce hazardous wastes, necessitate specialised equipment and high energy input, as well as heating during enzyme hydrolysis, and give rise to impure products (50-55% xylo-oligosaccharides) with xylose contamination. Indeed, a person skilled in the art knows that the use of high temperatures and/or pressures in these processes could be also expected to give rise to unwanted by-products such as furfural etc.

In contrast, in document WO 2009/117790, a multi-enzyme based multi-step process was described for xylo-oligosaccharide production from a commercial wheat bran. The pre-prepared, milled and sifted wheat bran was subjected to thermostable α-amylase treatment for 90 minutes at 90 °C to hydrolyse the starch, pH adjustment to pH 6 using concentrated HCl, protease treatment for 4 hours at 50 °C, pH 6, to remove residual protein (optional), boiling, filtration and washing, followed by multiple endoxylanase treatments for 10 h at 50 °C (glycoside hydrolase family 11 xylanase) and 1 h at 30 °C (glycoside hydrolase family 10 xylanase). Following enzyme inactivation by boiling for 30 minutes, the xylo-oligosaccharide solution was then concentrated, dried and further purified by ion exchange chromatography over a strong acid cation exchange resin and weak base anion exchange resin. Such a process, involving numerous successive steps and utilising numerous different enzymes with specific hydrolysis conditions, and involving complex processes such as chromatography requiring specific equipment and expertise, would be characterised by high costs and complexity.

In all of the above cases with land plant sourced lignocellulosic feedstocks, branched and/or non-branched homolinked β1,4 xylan and xylo-oligosaccharides were prepared.

Homolinked β1,3 and mixed linkage β1,3/β1,4-linked xylan have also been described in the state of the art, mainly in various green and red macroalgae, e.g., *Caulerpa, Bryopsis, Bangia, Porphyra,* and *Palmaria spp.*

Specifically, Maeda et al, 2012 described the isolation of homolinked β1,3 xylan and xylo-oligosaccharides from the green macroalgae *Caulerpa lentillifera* with a multi-step process involving: drying, grinding, high temperature alkaline treatment, acid treatment, NaClO$_4$ bleaching, alkaline extraction, ethanol precipitation and acetic acid washing, followed by xylo-oligosaccharides preparation by weak acid treatment at high temperature and pressure. This method suffers from having numerous steps, involving use of numerous hazardous chemicals and physical processes with high energy consumption, poor environmental impact and production of hazardous wastes, and requiring specialized equipment and specialized technical staff in addition to being characterised by challenges to process scale-up.

A similar multi-step procedure with similar environmental and economic challenges was described in JP 2001 086 999 for the closely related green macroalgae *Caulerpa racemosa* but, following xylan isolation using similar physical-chemical methods, final hydrolysis for xylo-oligosaccharide production involved use of a β1,3-xylanase at pH 6 and 37 °C. Thereafter, following reaction termination by heating at 100 °C for 5 minutes, the solution is treated with ethanol to remove large molecular weight xylo-oligosaccharides, centrifuged, and desalted.

Analogous to the xylo-oligosaccharides isolation procedures described for land-based plants, these processes described for preparation of homolinked β1,3 xylo-oligosaccharides from marine biomass also present many environmental and economic challenges. In addition, final xylo-oligosaccharides yields of as low as 5% were reported.

In addition to homolinked β1,3 and homolinked β1,4 xylans, mixed linkage β1,3/β1,4 xylan has also been described, namely in the red macroalgae *Palmaria palmata*. *Palmaria palmata* (also known as dulse, dillisk or dilsk) is a red macroalgae commonly found on the northern coasts of the Atlantic and Pacific Oceans. It is a fast growing, macroscopic, marine autotroph. Interestingly, notwithstanding a high xylan content and an absence of lignin, it has apparently been overlooked as a feedstock for xylan and xylo-oligosaccharide production. Furthermore, it is an abundant and carbon neutral, renewable marine resource which can be cultivated under controlled aquaculture conditions and can reduce strains on the world's resources (e.g. on food crops, arable land and freshwater), while also reducing pesticides and fertiliser use.

Interestingly, most studies on *Palmaria palmata* xylan have focused on its structural makeup, rather than its use as a source of novel xylo-oligsaccharides. In a study investigating the structure of the cell wall xylan of this macroalgae, Deniaud et al, 2003 described the isolation of impure heterolinked β1,3/β1,4 xylan. The isolation process involved initial preparation of an alcohol insoluble fraction (prepared by ethanol boiling, filtration, repeated ethanol, acetone, and chloroform:methanol treatments and drying), followed by sequential extraction „with, for example, saline, alkaline, and/or chaotropic agents (urea, guanidium thiocyanate) treatment (Deniaud et 2003). This process, involving use of various chemicals and solvents, suffers from a high complexity and associated high cost, a production of toxic wastes and pollutants, poor scale-up possibilities and a poor environmental footprint.

Similarly, Yamamoto et 2019 also used a complex, multi-step process for production of xylan, but also xylo-oligosaccharides, from a related Japanese *Palmaria sp*. The macroalgae was lyophilised and ground to a powder, suspended in 50-volumes of chloroform-methanol with stirring for 30 minutes, filtered and again mixed in chloroform-methanol, re-filtered, treated twice with 20-volumes of acetone, dried, resuspended in 40-volumes of water and autoclaved at 121 °C for 20 minutes. Xylan was then extracted by 8M urea treatment for 24 hours at room temperature, filtration, and dialysis against water before centrifugation and lyophilisation. This xylan rich fraction was hydrolysed to various xylo-oligosaccharides and xylose by xylanase treatment for 24 hours at 50 °C, pH 4.5, followed by heating to 100 °C for 5 minutes to stop the reaction. Such a complex, multi-step process making use of hazardous chemicals and high temperatures and pressures does not lend itself to safe and economically and environmentally viable xylan and xylo-oligosaccharides production. In addition, only 33.8% of the original xylan in the macroalgae was extracted to give a xylan-rich fraction which was only 52.2% pure. Similarly, only 66.6% of the xylan in the xylan rich fraction was converted to xylo-oligosaccharides which also contained undesired side products such as xylose and glucose.

A person skilled in the art would understand that, although numerous technologies have been described for xylan and xylo-oligosaccharides production and purification from various biomass sources, most present various economic and environmental challenges. These challenges are related to high costs and complexity associated with use of multiple steps, costly chemical use and recovery, requirements for specialised equipment, excess water use, high energy consumption, requirements for multiple enzymes, use of inefficient enzymes requiring specific hydrolysis conditions (temperature, pH etc.), waste production, challenges to process scale-up, costly downstream purification requirements, and production of undesired side products, breakdown and dehydration products (e.g. xylose, furfural, hydroxymethylfurfural), pollutants and inhibitors.

The identification of simplified procedures as described herein, avoiding the use of toxic/corrosive/hazardous/non-specific/costly chemicals and processes and/or multiple enzymes requiring specific energy inefficient reaction conditions would be of obvious advantage in the production of xylan and xylo-oligosaccharides.

These facts are disclosed in order to illustrate the technical problem addressed by the present disclosure.

## GENERAL DESCRIPTION

The present disclosure relates to a process for obtaining xylo-oligosaccharide (or xylo-oligomers) from an algae, comprising the following steps: pre-treating the algae to obtain algae flakes with a size ranging from 1-10 mm; preferably 1.5 - 4 mm; fractioning the algae flakes by aqueous extraction, to obtain a soluble fraction and an insoluble fraction, wherein the soluble fraction comprises a soluble xylan; hydrolysing the soluble fraction with 0.002 mg/L to 1 mg/L of endoxylanase, preferably at 15 to 30 °C, to obtain xylo-oligosaccharides, preferably xylo-oligosaccharides comprising mixed-link β1,3/β1,4 and homo-linked β1,4 xylo-oligosaccharides with a degree of polymerisation of at least 2-3; wherein the algae flakes comprise, on a dry weight basis: 30-50% (w/w) of xylan, 15-25% (w/w) of protein, 20-30% (w/w) ash, 2-10% (w/w) of lipids, 1-10% (w/w) of glucose, 3-15% (w/w) of galactose, 0.1-0.5% (w/w) of phenolics. Surprisingly, the obtained xylo-oligosaccharides are free of xylose contamination, as no xylose production was observed.

The size of the flakes can be measured by different methods. In the present disclosure the flakes' size was obtained by sieving the sample.

In an embodiment, the algae flakes comprise, on a dry weight basis: 35-45% (w/w) of xylan; 20-25% (w/w) of protein, 20-25% (w/w) ash; 3-8% (w/w) of lipids; 1-5% (w/w) of glucose; 3-8% (w/w) of galactose; and 0.2-0.3% (w/w) of phenolics.

In an embodiment, the concentration of endoxylanase ranges from 0.002mg/L to 0.1 mg/L; preferably 0.002 mg/L to 0.01 mg/L.

In an embodiment, the fractioning and hydrolysing steps are performed simultaneously.

In an embodiment, the soluble xylan is mixed-link β1,3/β1,4 xylan.

In an embodiment, the algae is an aquatic algae, preferably a marine algae, more preferably a cold water algae, even more preferably an algae from the northern Atlantic Ocean. In a further embodiment, the algae is a macroalgae.

In a further embodiment, the algae class is selected from a group comprising *Cyanidiales, Porphyridialies, Bangiales, Acrochaetiales, Betrachospermales, Nemaliales, Corallinales, Palmariales, Gelidiales, Gracilariales,* or *Ceramiales*; preferably the algae species is selected from a group comprising *Palmaria palmata, Palmaria georfica, Palmaria decipiens, Palmaria marginicrassa, Palmaria hecatensis, Palmaria mollis, Palmaria stenogona, Palmaria moniliformis, or Palmaria callophylloides*; more preferably the algae species is *Palmaria palmata*.

In an embodiment, the aqueous extraction is carried out with 10-200 g/L of algae flakes, dried and milled, suspended in distilled water, freshwater, sea water or salt water, for 1 to 12 hours, at a temperature ranging from 15 to 100 °C; preferably the aqueous extraction is carried out with agitation up to 120 rpm for 6 hours, at 15 - 25 °C.

In an embodiment, at least 50% (w/w) of the total algae's xylan is extracted to the soluble fraction, preferably 60 to 80% (w/w). In another embodiment, 20-40% (w/w), preferably 30-40% of the weight of the dried algae is extracted to the soluble fraction as xylan.

In an embodiment, the soluble fraction comprises, preferably consists of, on a dry weight basis: 50-70% (w/w) xylan, preferably 60-70% (w/w) xylan; up to 15% (w/w) galactose, preferably up to 10 % (w/w) galactose, even more preferably up to 5% (w/w) galactose; 1-5% (w/w) glucose, 20-25% (w/w) ash, 3-6% (w/w) protein and 0.4-0.8% (w/w) phenolics.

In an embodiment the disclosed process further comprises a step of ultrafiltration and/or dialysis of the soluble fraction to remove low molecular weight compounds.

In an embodiment the insoluble fraction comprises, on a dry weight basis: 45-55% (w/w) of protein; 10-20% (w/w) of xylan; 20-30% (w/w) ash; 0.1-0.5 % (w/w) of phenolics; and 7-10% (w/w) of lipids; wherein the protein is 70-90% (w/w) of the total protein found in the processed algae, i.e., on the algae flakes.

In an embodiment, the endoxylanase is a glycoside hydrolase family 8 or 11 endoxylanases (CAZy classification), preferably a glycoside hydrolase family 8 endoxylanase from *Pseudoalteromonas haloplanktis* TAH3a (UniProtKB Accession number - Q8RJN8 (Q8RJN8_PSEHA)).

The present disclosure also relates to a soluble xylan composition obtainable by the disclosed process comprising mixed-link β1,3/β1,4 xylan.

The present disclosure relates to a xylo-oligosaccharide composition obtainable by the disclosed process comprising xylo-oligosaccharides comprising mixed-link β1,3/β1,4 and homo-linked β1,4 xylo-oligosaccharides with a degree of polymerisation of at least 2-3.

In an embodiment, the xylo-oligosaccharide composition may comprise, on a dry weight basis, 50-70% (w/ w) of xylo-oligosaccharides, preferably 60-70% (w/w); up to15% (w/w) galactose; 1-5% (w/w) glucose; 20-25% (w/w) ash; 3-6% (w/w) protein; and 0.4-0.8% (w/w) phenolics. In a further embodiment, thexylo-oligosaccharide composition may comprise, on a dry weight basis, 50-70% (w/ w) of xylo-oligosaccharides, preferably 60-70% (w/w); 1-15% (w/w) galactose; 1-5% (w/w) glucose; 20-25% (w/w) ash; 3-6% (w/w) protein; and 0.4-0.8% (w/w) phenolics.

An aspect of the present disclosure relates to the use of the disclosed soluble xylan composition as food/feed products, in health products and health care, in materials preparation, platform chemicals production, biofuels production, cosmetics, research and development and/or as substrate for enzyme activity measurements and screening.

The present disclosure also relates to the use of the disclosed xylo-oligosaccharide composition as a prebiotic; as an anti-cancer agent; as an antioxidant; enhancer of organoleptic properties of foods, feeds, or beverages; in health care; as an immunostimulatory agent; anti-inflammatory agent; anti-microbial agent; as an enzyme substrate; as xylo-oligosaccharide standards in quantitative assays; as reagent in the production of biofuels, platform chemicals and bioplastics; or as cosmetic ingredient.

The present disclosure refers to a process for the production of xylo-oligosaccharides, xylan and/or protein-rich preparations from water biomass, preferably from flakes of a dried and milled algae. Specifically, said process is an eco-friendly process, based on aqueous extraction of washed, dried, and milled algae, and use of highly efficient enzymes highly active at low to moderate temperatures. The milled algae comprises, on a dry weight basis, 30-50% (w/w) of xylan, preferably 35-45% (w/w); 15-25% (w/w) of protein, preferably 20-25% (w/w); 20-30% (w/w) ash, preferably 20-25% (w/w); 2-10% (w/w) of lipids, preferably 3-8% (w/w); 1-10% (w/w) of glucose, preferably 1-5% (w/w); 3-15% (w/w) galactose, preferably 3-8% (w/w); and 0.1-0.5% (w/w) phenolics, preferably 0.2-0.3% (w/w); and is sieved to select dried flakes with 1 to 10mm, preferably 1.5 to 4mm.

More particularly, it is disclosed a process for preparing an oligosaccharide mixture which avoids the need for the currently used multi-step, expensive and hazardous processes and allows for the development of an environmentally friendly, sustainable, green chemistry production process. Additionally, the process enables for the simplified preparation of a soluble, mixed-link β1,3/β1,4 xylan rich fraction, and an insoluble protein rich fraction, which can have numerous potential applications in several industrial fields. In general lines, the steps of the process developed comprise *Palmaria palmata* washing and a pre-treatment involving thermal drying and milling to enable for high yielding, efficient aqueous extraction of a xylan rich soluble fraction and a protein-rich insoluble fraction, and the use of an endoxylanase enzyme, preferably a highly active, low temperature adapted endoxylanase, for highly efficient xylo-oligosaccharides preparation.

Furthermore, the present disclosure refers to a purified composition comprising a biomass solution, such as a marine biomass derived composition. More specifically, the composition comprises mixed-linkage β1,3/β1,4 and/or homo-linked β1,4 xylo-oligosaccharides from a red algae to be used in several applications. Particularly, the disclosed composition can be used in various industries, such as in food/feed, healthcare, research and development (R&D), materials, pharmaceutical, chemical, biofuels and/or cosmetic applications. The multi-valorisation of macroalgae through isolation and valorisation of both the xylan and protein fractions will obviously enhance its economic value while also contributing to the circular economy concept by valorising all major algal components and reducing wastes.

For the scope and interpretation of the present disclosure "marine biomass" is referred as a eukaryotic photosynthetic organism, selected from the group of *Archaeplastida*.

The present disclosure relates to a process for the production of xylan and xylan products from marine biomass, comprising aqueous extraction from washed, thermal dried and milled algae at a mesh size of 1.0-10 mm, and hydrolysis with highly active enzymes.

In an embodiment the products are: soluble xylan, an insoluble protein-rich fraction, and xylo-oligosaccharides comprising a mix of both mixed-link β1,3/β1,4 and homo-linked β1,4 xylo-oligosaccharides.

In an embodiment, the marine biomass is a eukaryotic photosynthetic organism, selected from the group of *Archaeplastida.*

In an embodiment, the marine biomass is an algae selected from the groups of *Chlorophyta* or *Rhodophyta,* which is selected from the group of *Cyanidiales, Porphyridialies, Bangiales, Acrochaetiales, Betrachospermales, Nemaliales, Corallinales, Palmariales, Gelidiales, Gracilariales, Ceramiales,* among others.

In an embodiment, the algae are selected from the family of *Palmariaceae* selected from the group of species: *Palmaria palmata, Palmaria georfica, Palmaria decipiens, Palmaria marginicrassa, Palmaria hecatensis, Palmaria mollis, Palmaria stenogona, Palmaria moniliformis, Palmaria callophylloides,* among others.

In an embodiment, the algae used is *Palmaria palmata* from the northern Atlantic Ocean or a cold-water species and it is wild harvested or cultivated by aquaculture.

In an embodiment, the drying and milling temperature is preferably between 20 and 120 °C and the mesh size is between 1.5 and 4 mm.

In an embodiment, the aqueous extraction uses distilled water, freshwater, sea water or salt water and is carried out with/without agitation for approximately 1-12 hours.

In an embodiment, the aqueous extraction is carried out with agitation at approximately 120 rpm for approximately 6 hours at a final concentration of approximately 100 g/L dried macroalgae.

In an embodiment, the aqueous extraction temperature is preferably approximately 15-25 °C or up to about 98 °C.

In an embodiment, the mixed-linkage β1,3/β1,4 xylan extracted in the soluble xylan-rich fraction represents 50-80%(w/w), preferably 60-80%(w/w), even more preferably greater than 65% (w/w), of the total xylan in *Palmaria palmata,* and 20-40%(w/w), preferably 30-40%(w/w), of the total dried macroalgae.

In an embodiment, the content of the soluble xylan-rich fraction is composed of, on a dry weight basis, 50-70% xylan (w/w), preferably 60-70% xylan (w/w), up to 15% (w/w) galactose, preferably less than 10% (w/w), even more preferably, less than 5% galactose (w/w), 1-5% glucose (w/w), 20-25% ash (w/w), 3-6% protein (w/w) and 0.4-0.8% phenolics (w/w). In a further embodiment, the soluble xylan-rich fraction is composed of, on a dry weight basis, 50-70% xylan (w/w), preferably 60-70% xylan (w/w), 1-15% (w/w) of galactose, 1-5% glucose (w/w), 20-25% ash (w/w), 3-6% protein (w/w) and 0.4-0.8% phenolics (w/w).

In an embodiment, ultrafiltration and/or dialysis is employed to remove low molecular weight compounds and the xylan content increases to, on a dry weight basis, 75-90% (w/w), preferably 80-90% (w/w), and the final xylan-rich product is concentrated and/or dried, or used in a liquid form.

In an embodiment, the insoluble fraction comprises, preferably consists of, 45-55% (w/w) protein, 10-20% xylan (w/w), 20-30% ash (w/w), 0.1-0.5 % phenolics (w/w) and 7-10% lipids (w/w), on a dry weight basis.

In an embodiment, the protein isolated in the insoluble protein rich fraction is, on a dry weight basis, 70-90% (w/w) of the total protein in *Palmaria palmata* and 20-25%(w/w) of the total dried macroalgae.

In an embodiment the xylo-oligosaccharides are produced from the isolated xylan-rich fraction or directly from a water suspension of the macroalgae by endoxylanase treatment.

In an embodiment, the content of xylo-oligosaccharides is, on a dry weight basis, 50-70% (w/w), preferably 60-70% (w/w), but can be increased to 70-90% (w/ w) by removal of low molecular components, e.g., ash, by ultrafiltration and/or dialysis.

In an embodiment the xylo-oligosaccharide composition may also contain up to 15% (w/w) galactose, 1-5% (w/w) glucose, 20-25% (w/w) ash, 3-6% (w/w) protein and 0.4-0.8% (w/w) phenolics. In a further embodiment, the xylo-oligosaccharide composition may also contain 1-15% (w/w) galactose, 1-5% (w/w) glucose, 20-25% (w/w) ash, 3-6% (w/w) protein and 0.4-0.8% (w/w) phenolics.

In an embodiment, the endoxylanase is preferably a glycoside hydrolase family 8 or 11 endoxylanase (CAZy classification).

In an embodiment the glycoside hydrolase used is a glycoside hydrolase family 8 endoxylanase from *Pseudoalteromonas haloplanktis* TAH3a (UniProtKB Accession number - Q8RJN8 (Q8RJN8_PSEHA)).

In an embodiment the xylo-oligosaccharide composition consists of both mixed linkage β1,3/β1,4 and homo-linked β1,4 xylo-oligosaccharides with an average degree of polymerisation of 4-7 and minimal degree of polymerisation of 2-3, without xylose monosaccharides production.

The present disclosure also relates to the use of the disclosed soluble xylan-rich fraction, the insoluble protein-rich fraction, and the xylo-oligosaccharides composition. The xylan-rich fraction can be used in the food/feed, health, materials, platform chemicals, biofuels, cosmetics, research and development (R&D) sectors and/or as substrate for enzyme activity measurements and screening. The insoluble protein-rich fraction can be used as a protein source in the food and feed industries or/and can be hydrolysed, or/and treated with solubilising agents, to produce soluble protein and/or peptides for the food, feed, and healthcare (e.g., bioactive peptides) industries. The xylo-oligosaccharide composition can be used as a prebiotic, enhancing short chain fatty acid production and/or Bifidobacteria growth; as an anti-cancer agent; as an antioxidant; in foods/feeds/beverages and other preparations for enhanced health benefits and/or organoleptic properties; in health care; as an immunostimulatory agent; as an anti-inflammatory agent; as an anti-microbial; as an enzyme substrate or xylo-oligosaccharides standards; in the production of biofuels and platform chemicals; in cosmetics; and in bioplastics production.

**BRIEF DESCRIPTION OF THE DRAWINGS**

The following figures provide preferred embodiments for illustrating the disclosure and should not be seen as limiting the scope of invention.

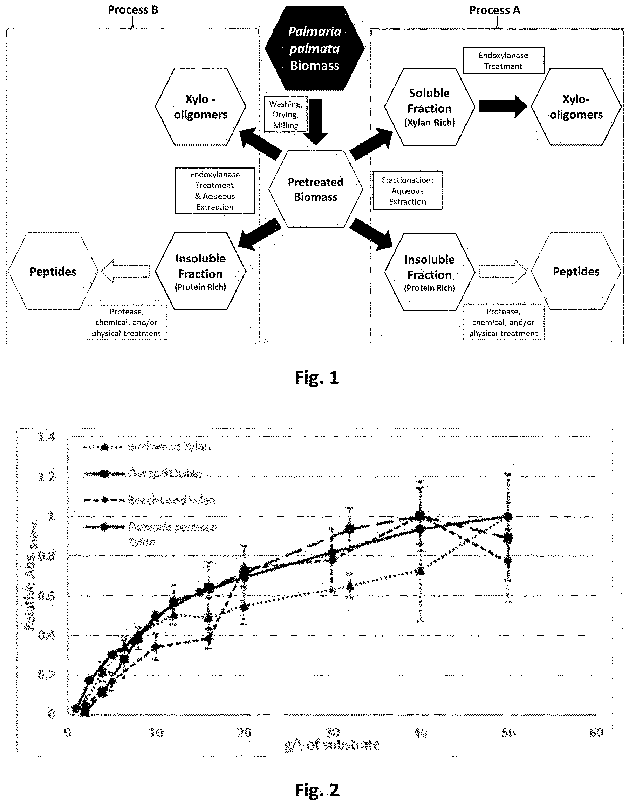

**Figure 1:** Illustrates a schematic of the processes for production of the xylan rich preparation, protein rich preparation and xylo-oligosaccharides preparation.

**Figure 2:** Comparison of the use of *Plamaria palmata* xylan-rich fraction, oat spelt xylan, birchwood xylan and beechwood xylan as substrates for DNS reducing sugar assays of cold-adapted glycoside hydrolase family 8 endoxylanase activity. Values of relative absorption readings at 546 nm for 3 independent tests, with three replicates each, at various substrate concentrations are shown.

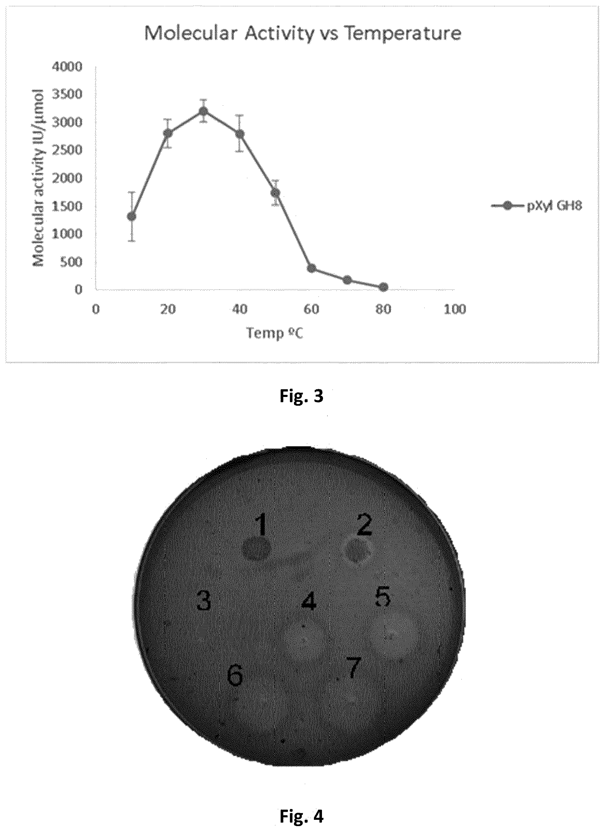

**Figure 3:** Illustrates the activity of the cold adapted glycoside hydrolase family 8 endoxylanse from *Pseudoalteromonas haloplanktis* TAH3a (UniProtKB Accession number: Q8RJN8 (Q8RJN8_PSEHA)) on the soluble xylan-rich fraction as a function of temperature. Activity is expressed as the molecular efficiency (μmol D-xylose reducing ends produced per minute per μmol of enzyme).

**Figure 4:** Illustrates the use of *Palmaria palmata* xylan-Remazol Brilliant Blue as a chromogenic substrate for xylanase activity screening. 1: negative control *(E. coli* BL21(DE3) cells); 2: *E. coli* BL21(DE3) cells expressing glycoside hydrolase family 8 (GH8) endoxylanase; 3: Blank (enzyme buffer: 20 mM MOPS + 100 mM NaCl); 4: 0.01 ug of GH8 xylanase; 5: 0.02 ug of GH8 xylanase; 6: 0.04 ug of GH8 xylanase; 7: 0.06 ug of GH8 xylanase.

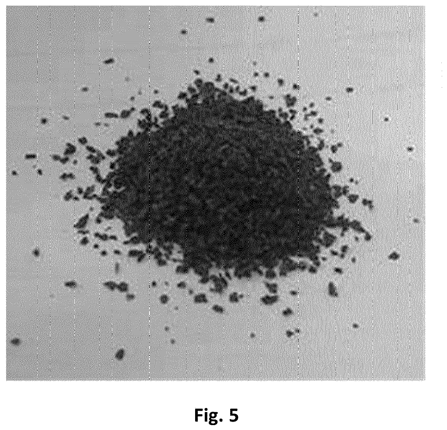

**Figure 5:** Embodiment of algae flakes obtained after the pre-treatment.

**DETAILED DESCRIPTION**

The present invention refers to a process for the production of xylo-oligosaccharides, soluble xylan and an insoluble protein-rich fraction from marine biomass, in particular algae. Specifically, said process is a simplified and eco-friendly process, based on aqueous extraction of washed, dried, milled algae and highly efficient enzymes, highly active at low to moderate temperatures. Surprisingly, the disclosed process resulted in a xylo-oligosaccharide composition free of xylose.

More particularly, it is disclosed a process for generating an oligosaccharide mixture which avoids the need for the currently used multi-step, expensive and hazardous processes and allows for the development of an environmentally friendly, sustainable, green chemistry production process. Xylo-oligosaccharide compositions comprising various mixed linkage β1,3/β1,4 and homolinked β1,4 xylo-oligosaccharides are prepared.

Additionally, the process enables for the simplified preparation of a soluble, mixed-link β1,3/β1,4 xylan rich fraction, and an insoluble protein rich fraction, associated with a low cost, efficient, high yield preparation, and which can have numerous potential applications in several industries.

For the purpose of the present disclosure, the algae used are a eukaryotic photosynthetic organism, selected from the group of *Archaeplastida.* Particularly, said algae are selected from the groups of *Chlorophyta* or *Rhodophyta.* More particularly, the algae are selected from the group of *Cyanidiales, Porphyridialies, Bangiales, Acrochaetiales, Betrachospermales, Nemaliales, Corallinales, Palmariales, Gelidiales, Gracilariales, Ceramiales,* among others. More particularly, said algae are selected from the family of *Palmariaceae* and type of Palmaria. More particularly, said algae is selected from the group of species: *Palmaria palmata, Palmaria georfica, Palmaria decipiens, Palmaria marginicrassa, Palmaria hecatensis, Palmaria mollis, Palmaria stenogona, Palmaria moniliformis, Palmaria callophylloides,* among others. In a preferable embodiment, the seaweed used is from the species of *P. palmata.* More particularly, the algae are *Palmaria palmata* from the northern Atlantic Ocean or a cold-water species.

The macroalgae can be wild harvested or cultivated by aquaculture using commonly employed cultivation procedures well known in the art. Macroalgae are subject to rapid microbial decomposition once harvested and hence rapid preservation is required, with drying to reduce water activity, and milling to reduce volume and weight, being commonly used. Nowadays, controlled reduced temperature air drying is most frequently applied to preserve heat labile components and product structure and sensory appeal.

In an embodiment, following aqueous washing, thermal drying and milling at high temperatures induced structural alterations and enabled simplified fractionation into a soluble xylan rich fraction and an insoluble protein rich fraction.

In general lines, following macroalgae cultivation and washing, the present process comprises a pre-treatment with a thermal drying and milling process for preparation of the algae, preferably *Palmaria palmata.* The cultivated algae are washed in water to remove epiphytes, sand and debris; thermal dried, milled and sieved with a mesh size of 1.0-10mm, preferably between 1.5 and 4 mm. The process temperature for thermal drying and milling, ranges between 20 °C to 120 °C, preferably 40 °C to 90 °C. The dried algae flakes, obtained after the pre-treatment, have a size between 1 to 10 mm, preferably 1.5 to 4 mm, and comprise on a dry weight basis, 30-50% (w/w) of xylan, preferably 35-45% (w/w); 15-25% (w/w) of protein, preferably 20-25% (w/w); 20-30% (w/w) ash, preferably 20-25% (w/w); 2-10% (w/w) of lipids, preferably 3-8% (w/w); 1-10% (w/w) of glucose, preferably 1-5% (w/w); 3-15% (w/w) galactose, preferably 3-8% (w/w); and 0.1-0.5% (w/w) phenolics, preferably 0.2-0.3% (w/w).

Following pre-treatment, the resulting dry algae flakes are subjected to an aqueous extraction. to give a xylan-rich soluble fraction and a protein-rich insoluble fraction. For highly efficient xylo-oligosaccharides preparation, endoxylanase enzyme treatment, either directly on an aqueous suspension of the pre-treated algae flakes or on the soluble xylan extract following separation, is carried out, preferably with a highly active, low temperature adapted endoxylanase with suitable product profile, in particular an absence of xylose production.

In a preferable embodiment of the present disclosure, a pre-treatment with washing, drying and milling of *Palmaria palmata* at high temperatures, preferably 40 °C to 90 °C leads to structural modifications of the macroalgae, such as precipitation of the protein and breakdown of physical interactions that hold the xylan in the algae structure, and enables high yield aqueous extraction into two fractions: (i) a soluble mixed-link β1,3/β1,4 xylan rich fraction and (ii) an insoluble protein rich fraction.

A schematic of an embodiment of the processes for the multi-valorisation of *Palmaria palmata* is shown in Figure 1. Novel xylo-oligosaccharide compositions and xylan-rich and protein-rich preparations can be efficiently produced with these processes. Following macroalgae pre-treatment and aqueous suspension, either of two processes can be employed: Process A: aqueous extraction and separation of soluble xylan-rich and insoluble protein-rich products followed by xylo-oligosaccharides production from the xylan-rich fraction; or Process B: xylo-oligosaccharide production by direct enzyme treatment of the pre-treated macroalgae suspension.

The benefits/applications of the various preparations produced according to the present disclosure are associated with the structural, chemical and/or functional properties of the mixture of components comprised therein. Also, the disclosed process results in a composition of xylo-oligosaccharides free of xylose.

In an embodiment, after the pre-treatment of the marine biomass, the process comprises the following protocol:

Aqueous extraction involves suspending the dried pre-treated algae, preferably *Palmaria palmata,* in distilled water, freshwater, sea water or salt water (e.g., 3.5 wt.% NaCl). The suspension can be carried out with agitation for approximately 1-12 hours, or in another embodiment, the suspension is not submitted to agitation. In a preferable embodiment of the present disclosure, the aqueous extraction was carried out using fresh water, such as distilled water, with agitation for approximately 6 hours at a final concentration of approximately 100 g/L dried macroalgae. The temperature of the aqueous extraction is preferably the ambient temperature (between 15-25 °C), thereby reducing heating and energy requirements. In another embodiment, the aqueous extraction is carried out at high temperatures (up to about 98 °C) for enhanced yield but also higher contaminant extraction (protein and other components in the soluble xylan-rich fraction).

For the scope and interpretation of the present disclosure it is defined that "ambient temperature", or "room temperature", should be regarded as a temperature between 15-25 °C, preferably between 18-20 °C, more preferably 20 °C.

In an embodiment, the aqueous extraction can be performed using different concentrations of dried *Palmaria palmata,* preferably ranging between 10 - 200 g/L. In a further embodiment, the concentration of *Palmaria palmata* affects the reaction yield and total xylan content. Increasing concentrations of *Palmaria palmata* within this range (10- 200 g/L), leads to decreasing yields and increasing total xylan content.

In an embodiment, the incubation time may vary between 1 to 12 hours. Longer or shorter incubation times lead to lower yields at short incubation times (less than 1 hour) and little increase in yield at longer incubation times (greater than 12 hours). Preferably, agitation at 120 rpm is used during the incubation time. No agitation leads to lower xylan extraction yield. Incubation at ambient temperature reduces heating and energy costs and reduces the environmental impact.

The soluble fraction can be separated from the insoluble fraction by any kind of separation method known to a person skilled in the art. For example, but not limiting the scope of the invention, the methods which can be used are ultrafiltration, centrifugation, and/or pressing of the insoluble fraction, e.g., with a screw press, belt filter press, filter press; to enhance soluble fraction yield. In a preferable embodiment of the present disclosure the separation method was pressing and/or ultrafiltration.

In an embodiment, the extraction yield for the mixed-linkage $\beta1,3/\beta1,4$ xylan in the soluble xylan-rich fraction is greater than 50% (w/w) or greater than 65% (w/w), preferably between 50-80% (w/w), more preferably 60-80% (w/w) of the total xylan in *Palmaria palmata,* and 20-40% (w/w), preferably 30-40% (w/w), of the total dried macroalgae.

In an embodiment, the yields were calculated following equation 1 and equation 2, considering the total weight of xylan extracted per weight of xylan in the macroalgae, and the total weight of xylan extracted per weight of the dried algae, respectively.

$$ Yield\left(\frac{w}{w}\%\right) = \frac{\text{weight of xylan extracted}}{\text{weight of xylan in macroalgae}} \times 100 \qquad \text{Equation 1} $$

$$ Yield\left(\frac{w}{w}\%\right) = \frac{\text{weight of xylan extracted}}{\text{weight of dry macroalgae}} \times 100 \qquad \text{Equation 2} $$

In an embodiment, the soluble xylan-rich fraction obtained is composed, on a dry weight basis, of 50-70% (w/w) xylan, preferably 60-70% (w/ w) xylan, as well as up to 15% (w/w) galactose, preferably up to 10% (w/w) of galactose, even more preferably up to 5% (w/w) galactose, 1-5% (w/w) glucose, 20-25% (w/w) ash, 3-6% (w/w) protein and 0.4-0.8% (w/w) phenolics. In another embodiment, higher ash content, up to 60% (w/w), is observed when marine or salt water is used for fractionation.

In an embodiment, ultrafiltration and/or dialysis can be employed to remove low molecular weight compounds, e.g. ash, and thereby increase xylan content to 75-90% (w/w), preferably 80-90% (w/w), on a dry weight basis.

In an embodiment, the final xylan-rich product can be concentrated and/or dried, or used in a liquid form.

In contrast to currently available xylan products which are generally poorly soluble, the xylan obtainable by the disclosed process is soluble and absent of undesirable breakdown or side products and thereby holds the potential for simplifying and enhancing xylan application and opening up new valorisation routes.

The soluble xylan rich fraction produced can be utilised in the food/feed (e.g., flavouring, dietary fiber, feedstock for xylitol production), health (e.g. dietary fiber), materials (e.g., feedstock for bioplastics, polyhydroxyalkanoates production), platform chemicals (e.g., feedstock for 1,4 butanediol, furfural, xylonic acid, xylitol production), biofuels (feedstock for biofuels production), cosmetics (e.g., skin and hair care), and research and development (R&D) sectors (e.g., soluble xylanase substrate enabling improved assay reproducibility as compared to currently used insoluble xylanase substrates). A person skilled in the art knows that the use of xylan as feedstock for materials, platform chemicals and/or biofuels production requires the hydrolysis of the xylan to xylose monomers by physical, chemical, and/or enzymatic hydrolysis methods, either with purified/non-purified/partly purified enzymes and/or whole cells. For these purposes, xylan hydrolysis to xylose can be carried out before or simultaneous to the transformation to the higher value-added products. As an example, treatment of the xylan-rich fraction obtained by the process of the present disclosure with 4 wt.% sulphuric acid at 121 °C and 1 bar for 20 minutes enables efficient xylose preparation.

As a further example of application, the xylan produced by the disclosed process can be used in research and development (R&D) as a substrate for measurement of xylanase activity, by for example the dinitrosalicylic acid (DNS) and/or Nelson-Somogyi (NS) assays, and/or in screening for xylanase activity. The xylan produced by the disclosed process is highly soluble (maximum solubility up to 150 g/L) and, as shown in Figure 2, results in improved assay reproducibility as compared to other commonly used commercial xylans, e.g., beechwood xylan, birch wood xylan, and oat spelt xylan which are insoluble or only poorly soluble.

Concerning the insoluble fraction obtained from the macroalgae following the aqueous extraction process, it is protein rich, being composed of, on a dry basis, 45-55% (w/w) protein, 10-20% (w/w) xylan, 20-30% (w/w) ash, 0.1-0.5 % (w/w) phenolics and 7-10% (w/w) lipids. Extraction yields for the protein in the insoluble protein rich fraction are 70-90% (w/w) of the total protein in *Palmaria palmata* and 20-25%(w/w) of the total dried macroalgae. In an embodiment, this fraction can be utilised as a protein source in the food and feed industries or/and can be hydrolysed, e.g., with protease enzymes, or/and treated with solubilising agents, to produce soluble protein and/or peptides for the food, feed, and healthcare (bioactive peptides) industries.

In an embodiment, xylo-oligosaccharides can be produced from the isolated xylan-rich fraction, as shown in Figure 1, Process A, or directly from a water suspension of the pre-treated macroalgae, as shown in Figure 1, Process B, by direct endo-β-(1,4)-xylanase (also known as endoxylanase, EC 3.2.1.8) treatment of the aqueous solutions/suspensions.

In the case of direct xylo-oligosaccharide production from a *Palmaria palmata* pre-treated suspension (Figure 1, Process B), preferably, a concentration of 10-200 g/L of pre-treated dried macroalgae per litre of water is used, more preferably a concentration of 100 g/L is used. Freshwater, sea water, distilled water, or salt (e.g., 3.5 wt. % NaCl) added water can be used. Sea water and salt added water led to higher ash content in the fractions produced.

For both processes, A and B, endoxylanases, preferably glycoside hydrolase family 8 or 11 endoxylanases (CAZy classification) can be used to produce various linear mixed linkage β1,3/β1,4 and homolinkage β1,4 xylo-oligosaccharides. Most preferably, the glycoside hydrolase used is a glycoside hydrolase family 8 endoxylanase from *Pseudoalteromonas haloplanktis* TAH3a (UniProtKB Accession number - Q8RJN8 (Q8RJN8_PSEHA)), with high activity at low to moderate temperatures as shown in Figure 3 and Table I. In an embodiment, the enzyme was produced and purified using procedures described in Collins et al., 2002. Heterologous protein expression by batch production in rich media with *Escherichia coli* as host was used for recombinant xylanase production. A combination of anion exchange, cation exchange and gel filtration chromatography (GE Healthcare Life Sciences) was used for purification.

Particularly, 0.002 mg to 1 mg, preferably 0.002 mg to 0.1 mg, even more preferably 0.002 mg to 0.01 mg of endoxylanase is added per litre of the xylan-rich fraction or algal suspension. Most preferably, incubation is carried out at 170 rpm, for 12 hours. In an embodiment, endoxylanase hydrolysis can be carried at the optimum temperature of the enzyme employed, preferably at 15-30 °C and most preferably at ambient temperature, approximately 20 °C, with cold-adapted enzymes. In another embodiment, hydrolysis can be carried out at 50-60 °C, employing enzymes adapted to higher temperatures, such as those shown in Table I.

**Table I.** Activity of endoxylanases at room temperature (20 °C) and at the optimum temperature of activity.

| Enzymes | Molecular Activity (IU/μmol) at 20 °C) | Molecular Activity (IU/μmol) at Optimum Temp |
|---|---|---|
| GH8 Xylanase | 2799 | 3204 (30 °C) |
| Nzy8A (CpXyn8A) | 54 | 356 (60 °C) |
| Mega10A (E-XYNACJ) | 34 | 388 (60 °C) |
| Mega11A (E-XYLNP) | 318 | 2986 (60 °C) |
| Nzy11A (RfXyn11A) | 349 | 1906 (50 °C) |

\* Activity is expressed as the molecular efficiency (μmol D-xylose reducing ends produced per minute per μmol of enzyme). GH8 Xylanase is the cold adapted glycoside hydrolase family 8 endoxylanase from *Pseudoalteromonas haloplanktis* TAH3a (UniProtKB Accession number - Q8RJN8 (Q8RJN8_PSEHA)). Nzy8A(CpXyn8A) is a glycoside hydrolase family 8 endoxylanse from *Clostridium papyrosolvens* (NZYTech; CZ0915). Mega10A (E-XYNACJ) is a family 10 endoxylanase (Megazyme). Mega11A (E-XYLNP) is a glycoside hydrolase family 11 endxylanase (Megazyme). Nzy11A (RfXyn11A) is a glycoside hydrolase family 11 endoxylanase from *Ruminococcus flavefaciens* (NZYTech; CZ0325).

In a preferable embodiment of the disclosure, cold adapted endoxylanases, which are highly active at low to moderate temperatures such as ambient temperature, are employed to enable ambient temperature incubation with low concentrations of enzyme (such as 0.002 to 0.01 mg/L) and thereby enabling reduced heating, reduced energy requirements, reduced environmental impact, reduced enzyme amount, and consequently reduced costs.

In an embodiment, family 8 or 11 mesophilic endoxylanases (adapted to higher temperatures) can also be used at ambient temperatures but are less active. Heating to the optimal temperature of activity (about 50-60 °C) enables for enhanced hydrolysis efficiency (see Table I for activity measurements at 20 °C and at the maximum temperature of each enzyme tested) but with negative economic and environmental impact.

Preferably, temperatures between 15-60 °C are used. Even more preferably, ambient temperature incubation is used for the cold-adapted family 8 endoxylanase. Preferably mixing is employed. The incubation time is dependent on the enzyme used, enzyme concentration, substrate concentration and incubation temperature. Most preferably, incubation times of between 3 and 24 hours are used, more preferably 3 to 15 hours, even more preferably 12 hours of incubation is used with the cold-adapted family 8 endoxylanase. Ideally, conditions should enable for completion of the substrate hydrolysis process to give the final xylo-oligosaccharides products. As is evident to those practised in the art, shorter incubation times can be achieved by use of higher enzyme concentrations, within the given concentration range.

In an embodiment, the final xylo-oligosaccharide composition consists of both mixed linkage $\beta1,3/\beta1,4$ and homolinked $\beta1,4$ xylo-oligosaccharides. An average degree of polymerisation of 4-7 and minimal degree of polymerisation of 2-3 is preferred, most preferably 3. Most preferably, xylose monosaccharides are not produced, in contrast to many currently used xylo-oligosaccharide production processes which often yield monosaccharides and other contaminants or/and breakdown products.

In an embodiment, the xylo-oligosaccharide content is 50-70% (w/w), preferably 60-70% (w/w), but can be increased to 70-90% (w/w) by removal of low molecular components, e.g., ash, by ultrafiltration and/or dialysis. The composition may also contain up to 15% (w/w) of galactose, 1-5% (w/w) glucose, 20-25% (w/w) ash, 3-6% (w/w) protein and 0.4-0.8% (w/w) phenolics. In a further embodiment, the composition may also contain 1-15% (w/w) galactose, 1-5% (w/w) glucose, 20-25% (w/w) ash, 3-6% (w/w) protein and 0.4-0.8% (w/w) phenolics. The xylo-oligosaccharides can be fractionated according to size by, e.g, ultrafiltration.

In an embodiment, the final product can be concentrated by, e.g., ultrafiltration, and/or dried, or used in a liquid form.

The xylo-oligosaccharide composition thus prepared is particularly suited for use as a prebiotic (functional food, functional feed, functional beverage, functional preparation, functional additive, functional ingredient), enhancing short chain fatty acid production and/or Bifidobacteria growth. It is also suited for use as an anti-cancer agent, reducing the viability of cancer cells, and/or as an antioxidant. Further application include foods, feeds, beverages or other preparations for enhanced health benefits and/or organoleptic properties; health care (anti-cancer, immunostimulatory, anti-inflammatory, anti-microbial, and/or anti-oxidant agent); R&D as an enzyme substrate or as xylooligosaccharide standards; production of biofuels and platform chemicals (as a source of xylose); cosmetics (skin and/or hair care) and materials (e.g. bioplastics, polyhydroxyalkanoate). A person skilled in the art knows that the use of the xylooligosaccharides as feedstock for materials, platform chemicals and/or biofuels production requires the hydrolysis of the xylan to xylose monomers by physical, chemical, and/or enzymatic hydrolysis methods, either with purified/non-purified/partly purified enzymes and/or whole cells. For these purposes, xylo-oligosaccharides hydrolysis to xylose can be carried out before or simultaneous to the transformation into higher value-added products.

It must be understood that the examples and embodiments described herein are solely by way of illustration and that several modifications or changes, without departing from the spirit and scope thereof, in the light of themselves, will be apparent to those skilled in the art and must be included in the scope and spirit of this description and attached claims.

EXAMPLES

The following experimental examples illustrate the present invention, without, however, limiting the coverage of its scope. In general lines, the examples herein mentioned are the illustrative description of the applications of the products obtained from the production process above detailed.

Compositional Analysis: Methods

Compositional analysis and yield determination of the dried *Palmaria palmata* and the various preparations produced are carried out using techniques known to the art as described below:

Lipid content:

The lipid content was determined as described by Bligh & Dyer 1959. In summary, lipids were extracted using a water/chloroform/methanol mixture with a final ratio of 1.8/2/2. For dried samples, water was added to a water content of 80% (w/w) before extraction.

Ash content:

Ash content was analysed according to AACC Method 08-01.01, and Laurens et *al.* 2012. Samples were incubated at 550 °C for 16 hours in a muffle furnace (NABERTHERM LVT 15/11) and weighed.

Moisture content:

Moisture content was measured as described by Ahn *et al.* 2014. Weighed samples were dried overnight in ceramic crucibles in a drying oven (80 °C) and re-weighed.

Phenolic compounds:

Four consecutive extractions with distilled water at 98 °C, 180 rpm for 5 hours were pooled, and phenolic levels quantified as described by Machu *et al.* 2015.

Protein content:

Soluble protein content was determined using the Lowry method as described by Waterborg *et al.* 2003, based on the reaction of the peptide bonds of proteins with copper under alkaline conditions, and reaction with the Folin reagent.
Nitrogen/protein content in insoluble samples was determined by the Kjeldahl method. In summary, samples were hydrolysed with $H_2SO_4$ at 550 °C for 30 minutes in a FOSS Labtec DT 208 Digestor before distillation in the FOSS KJELTECTM 8400 Analyser Unit. Protein content was determined using a conversion factor of 6.25.

Carbohydrate content:

Carbohydrate content was determined by HPLC analysis of hydrolysed material. 4% (w/v) $H_2SO_4$ treatment at 121 °C, 1 bar for 20 minutes hydrolysed carbohydrates to monomers which were then separated and quantified by HPLC with a ROA-organic acid H+(8%) column (Phenomenex). 2.5 mM $H_2SO_4$ was used as mobile phase at a flow rate of 0.7 mL/min for the first 7 minutes followed by 0.1 mL/min for a total period of 30 minutes. Standards curves with various concentrations of xylose and glucose were used for concentration determination.

Galactose content:

Galactose content was determined with the L-Arabinose/D-Galactose Assay Kit as described by the manufacturers (Megazyme).

Xylo-oligosaccharides identification:

Xylo-oligosaccharides were separated and identified by HPLC analysis on a Rezex RSO-Oligosaccharide Ag+ (4)% column (Phenomenex) and comparison to standard commercial β1,4 xylo-oligosaccharides. Water was used as mobile phase at a constant flow rate of 0.15 mL/min for a total period of 90 minutes.
Molecular weight distributions were analysed with a Rezex PolySep-SEC GFC-P Linear (Phenomenex) and Biobasic SEC 60 HPLC Columns as described by the manufacturers.
The Nelson-Somogyi (NS) and 3,5-dinitrosalicylic acid (DNS) assays, well known to those skilled in the art, were used to calculate the carbohydrate reducing ends content of samples as well as for enzyme activity measurements (McCleary and McGeough, 2015).

Applications

Xylan as Substrate for Enzyme Activity Measurements

In an embodiment of the use of the *Palmaria palmata* xylan-rich fraction as an advantageous substrate for reproducible and accurate measurement and detection of endoxylanase activity, its use in the DNS and NS activity assays, and as a chromogenic substrate for activity detection in a petri-dish solid media plate-assay were investigated.
For the DNS and NS assays (McCleary and McGeough, 2015), measurements of the glycoside hydrolase family 8 cold-adapted β1,4-xylanase activity were made over a range of substrate concentrations with various substrates: oat spelt xylan (Sigma), birchwood xylan (Sigma), beechwood xylan (Carl Roth) and the *Palmaria palmata* xylan-rich fraction obtained by the disclosed process. Surprisingly, as can be seen from Figure 2 for the DNS assay, and probably resultant of its high solubility, the *Palmaria palmata* xylan enables a much higher assay precision (average coefficient of variation for all tests over all substrate concentrations tested = 4%) and accuracy for measurements as compared to all other substrates tested. Such assay precision and accuracy can be related to the high solubility of the *Palmaria palmata* xylan obtained by the process herein disclosed. Oat spelt, birchwood and beechwood xylans are poorly soluble in water and activity assays are characterised by a poor precision, which negatively affects test accuracy, in particular at high substrate concentrations. Average coefficients of variation of 21%, 21% and 15% were observed for oat spelt, birchwood and beechwood xylans, respectively. This was found to increase significantly at substrate concentrations greater than ~30 mg/mL, where increased viscosity and coefficients of variation as high as 36%, 54% and 27% were observed for oat spelt, birchwood and beechwood xylan, respectively. The observed enhanced assay precision for the disclosed *Palmaria palmata* xylan, as compared to the other xylan substrates tested, points to its high suitability for use as assay substrate. Furthermore, in a demonstration of its widespread applicability for activity assays of various types of xylanases, the substrate was found to enable measurement of activities of endoxylanases belonging to various glycoside hydrolase families, including β1,4 xylanases of families 8, 10 and 11, and β1,3 xylanases of family 26.

Xylan as Substrate for Enzyme Activity Screening

The use of xylan for solid media plate-screening of xylanases was demonstrated by coupling with the anthraquinone dye remazol brilliant blue as described by Biely *etal.,* 1985 and incorporation at a final concentration of 0.1% into Luria Bertani agar media (5 g/L yeast extract, 10 g/L bacto-tryptone, 5 g/L NaCl and 20 g/L agar). As shown in Figure 4, a clear halo is observed around endoxylanase producing colonies and around wells containing endoxylanase solutions whereas no halos are observed in the absence of xylanase activity. Such results clearly point to the potential of this substrate for use in detecting and screening for xylanase activity.

Xylo-oligosaccharides as Prebiotics

In an embodiment of the use of the xylo-oligosaccharides preparation(s) of the present disclosure is their use as a prebiotic for human and animal health and well-being. The prebiotic potential of the xylo-oligosaccharides can be assessed and compared to negative controls without prebiotic addition by *in vitro* fermentation studies with human faecal inocula. Key markers of gastrointestinal health such as the production of short chain fatty acids (acetate, propionate, butyrate, etc.), lactate, ammonia and gas ($H_2$, $CO_2$), as well as changes in microbiota composition, are monitored by HPLC, GC and high-throughput sequencing as previously described (Amorim *et* 2020). Xylo-oligosaccharides addition leading to significant increases in the total production of short chain fatty acids and lactate, important metabolites with numerous health benefits, as well as a modulation of microbiota composition towards a decreased abundance of Proteobacteria, these being associated with several intestinal diseases, points to a potential as prebiotics.

Xylo-oligosaccharides as Anticancer Agents

In a further embodiment, the reduction of the viability of mammalian breast cancer and colorectal cancer cells (such as MCF-7 cell line, or HCT-116 cell line) by xylo-oligosaccharides in a dose-dependent manner, points to application as an anticancer agent. Measurements of anticancer activity can be carried out using assays well known to those skilled in the art, namely the colorimetric microculture tetrazolium assay (MTT) for monitoring metabolic activity, and the acridine orange (AO) and propidium iodide (PI) double staining assay for monitoring membrane integrity and cell viability. Significant reductions in cancer cell proliferation as compared to negative controls while not affecting normal cell viability points to potential as anticancer agents.

Xylo-oligosaccharides as Anti-Oxidants

In yet another embodiment, the anti-oxidant activity of the xylo-oligosaccharide preparation(s) can be demonstrated by the commonly used DCF reactive oxygen species assay, employed to measure reactive oxygen species (ROS) activity within cells. Mammalian cells, with and without stimulation, can be incubated in the absence and presence of various concentrations of xylo-oligosaccharides, stained with 2',7'-dichlorodihydrofluorescin diacetate, and fluorescence measured with excitation at 485 nm and emission at 538 nm. A significantly decreased fluorescence measurement for xylo-oligosaccharides treated cells as compared to controls indicates a significant reduction in the levels of reactive oxygen species (ROS) and an anti-oxidant activity.

Xylan and Xylo-oligosaccharides for Xylose

The relatively pure xylan-rich fraction and xylo-oligosaccharides mix produced herein, without breakdown products, chemicals or unwanted side products, can be used as feedstocks for xylose in the preparation of various high value products, e.g., materials (e.g., bioplastics, polyhydroxyalkanoates), platform chemicals (e.g., 1,4 butanediol, furfural, xylonic acid, xylitol) and biofuels. The xylan and xylo-oligosaccharides can be hydrolysed to xylose by physical, chemical, and/or enzymatic hydrolysis methods, either with purified/non-purified/partly purified enzymes and/or whole cells. In the present invention, xylose was efficiently produced, without breakdown products such as furfural etc., by treatment of the xylan-rich fraction, and/or xylo-oligosaccharides, with 4 wt. % sulphuric acid at 121 °C and 1 bar for 20 minutes.

All the publications and patent applications mentioned in the description are indicative of the level of those skilled in the art to which this invention relates to. All publications and patent applications are herein incorporated by reference to the same extent as each individual publication or each patent application were specifically and individually indicated to be incorporated by reference.

Despite the invention having been described in some detail by way of illustration and examples for a matter of clarity and understanding, it will be evident that certain changes and modifications can be practiced within the scope of the attached claims of this description.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above-described embodiments are combinable.

The following claims further set out particular embodiments of the disclosure.

BIBLIOGRAPHY

Aachary, A.A., Prapulla, S.G., 2011. Xylooligosaccharides (XOS) as an Emerging Prebiotic: Microbial Synthesis, Utilization, Structural Characterization, Bioactive Properties, and Applications. Compr. Rev. Food Sci. Food Saf. 10, 2-16. https://doi.org/10.1111/j.1541-4337.2010.00135.

Ahn, J.Y., Kil, D.Y., Kong, C., Kim, B.G., 2014. Comparison of oven-drying methods for determination of moisture content in feed ingredients. Asian-Australasian J. Anim. Sci. 27, 1615-1622. https://doi.org/10.5713/ajas.2014.14305.

Amorim, C., Silvério, S.C., Prather, K.L.J., Rodrigues, L.R., 2019. From lignocellulosic residues to market: Production and commercial potential of xylooligosaccharides. Biotechnol. Adv. 37, 107397. https://doi.org/10.1016/j.biotechadv.2019.05.003 .

Ávila, P.F., Franco Cairo, J.P.L., Damasio, A., Forte, M.B.S., Goldbeck, R., 2020. Xylooligosaccharides production from a sugarcane biomass mixture: Effects of commercial enzyme combinations on bagasse/straw hydrolysis pre-treated using different strategies. Food Res. Int. 128, 108702. https://doi.org/10.1016/j.foodres.2019.108702 .

Biely, P., Mislovicova, D., Toman, R., 1985. Soluble chromogenic substrates for the assay of endo-1,4-beta-xylanases and endo-1,4-beta-glucanases. Anal. Biochem. 144, 142-146.

Bligh, E.G., Dyer, W.J., 1959. A rapid method of total lipid extraction and purifcation. Can. J. Biochem. Physiol. 37, 911-917. https://doi.org/10.1139/o59-099.

Broekaert, W., Courtin, C., Delcour, J., 2009. (Arabino)xylan oligosaccharide preparation. WO 2009/117790 A2.

Collins, T., Gerday, C., Feller, G., 2005. Xylanases, xylanase families and extremophilic xylanases. FEMS Microbiol. Rev. 29. https://doi.org/10.1016/j.femsre.2004.06.005

Collins, T., Meuwis, M.A., Stals, I., Claeyssens, M., Feller, G., Gerday, C., 2002. A novel family 8 xylanase, functional and physicochemical characterization. J. Biol. Chem., 277, pp. 35133-35139. https://www.jbc.org/action/showPdf?pii=S0021-9258%2818%2936742-5

Courtin, C.M., Swennen, K., Verjans, P., Delcour, J.A., 2009. Heat and pH stability of prebiotic arabinoxylooligosaccharides, xylooligosaccharides and fructooligosaccharides. Food Chem. 112, 831-837. https://doi.org/10.1016/j.foodchem.2008.06.039

Delcour, J., Courtin, C., Broekaert, W., Swennen, K., Verbeke, K., Ruteers P, 2006. Prebiotic preparation. WO 2006/002495 A1.

Deniaud, E., Fleurence, J., Lahaye, M., 2003. Interactions of the mix-linked beta-(1,3)/-(1,4)-d-xylans in the cell walls of Palmaria palmata (Rhodophyta). J. Phycol. 39, 74-82. https://doi.org/10.1046/j.1529-8817.2003.02047.x

Hsu, C.K., Liao, J.W., Chung, Y.C., Hsieh, C.P., Chan, Y.C., 2004. Xylooligosaccharides and fructooligosaccharides affect the intestinal microbiota and precancerous colonic lesion development in rats. J Nutr 134, 1523-1528. https://doi.org/10.1093/jn/134.6.1523

Ito, M., Yamaguchi, K., Izumi, Y., 2001. Composition containing xylooligosaccharide and carcinoma cell apoptosis inducer. JP 2001086999.

Jiang, J., You, Y., Zhang, W., Yang, S., Zhu, L., 2018. A kind of preparation method of xylo-oligosaccharide. CN108251472A.

Kabel, M.A., Kortenoeven, L., Schols, H.A., Voragen, A.G.J., 2002. In vitro fermentability of differently substituted xylo-oligosaccharides. J. Agric. Food Chem. 50, 6205-6210. https://doi.org/10.1021/jf020220r

Kim, M.-J., Yoo, S.-H., Jung, S., Park, M.-K., Hong, J.-H., 2015. Relative Sweetness, Sweetness Quality, and Temporal Profile of Xylooligosaccharides and Luo Han Guo (Siraitia grosvenorii) Extract. Food Sci. Biotechnol 24, 965-973. https://doi.org/10.1007/s10068-015-0124-x

Laurens, L.M.L., Dempster, T.A., Jones, H.D.T., Wolfrum, E.J., Van Wychen, S., McAllister, J.S.P., Rencenberger, M., Parchert, K.J., Gloe, L.M., 2012. Algal biomass constituent analysis: method uncertainties and investigation of the underlying measuring chemistries. Anal. Chem. 84, 1879-1887. https://doi.org/10.1021/ac202668c

Machu, L., Misurcova, L., Vavra Ambrozova, J., Orsavova, J., Mlcek, J., Sochor, J., Jurikova, T., 2015. Phenolic content and antioxidant capacity in algal food products. Molecules 20, 1118-1133. https://doi.org/10.3390/molecules20011118

Maeda, R., Ida, T., Ihara, H., Sakamoto, T., 2012. Induction of apoptosis in MCF-7 cells by $\beta$-1,3-xylooligosaccharides prepared from Caulerpa lentillifera. Biosci. Biotechnol. Biochem. https://doi.org/10.1271/bbb.120016

McCleary, B. V., McGeough, P., 2015. A comparison of polysaccharide substrates and reducing sugar methods for the measurement of endo-1,4-$\beta$-xylanase. Appl. Biochem. Biotechnol. 177, 1152-1163. https://doi.org/10.1007/s12010-015-1803-z

Shiyuan, Y., Hanjing, Z., Yong, X., Qiang, Y., Hansheng, Z., 2011. Improved method for preparing plant xylan through extraction by alkali method. CN101531722A.

Suwa, Y., Koga, K., Fujikawa, S., Okazaki, M., Irie, T., Nakada, T., 1992. Lactobacillus bifidus proliferation promoting composition. EP 0265970 B1.

Teleman, A., Lundqvist, J., Tjerneld, F., Stålbrand, H., Dahlman, O., 2000. Characterization of acetylated 4-O-methylglucuronoxylan isolated from aspen employing 1H and 13C NMR spectroscopy. Carbohydr. Res. 329, 807-815. https://doi.org/10.1016/S0008-6215(00)00249-4

Tsuji, S., Watanabe, M., Nuruki, Y., Miyawaki, S., 2017. Method for producing xylan-containing material. EP3252083A1.

Wang, Y., Zhang, J., 2006. A novel hybrid process, enhanced by ultrasonication, for xylan extraction from corncobs and hydrolysis of xylan to xylose by xylanase. J. Food Eng. 77, 140-145. https://doi.org/10.1016/j.jfoodeng.2005.06.056

Waterborg, J.H., Matthews, H.R., 2003. The Lowry method for protein quantitation, in: Basic Protein and Peptide Protocols. Humana Press, New Jersey, pp. 1-4. https://doi.org/10.1385/0-89603-268-X:1

Yamamoto, Y., Kishimura, H., Kinoshita, Y., Saburi, W., Kumagai, Y., Yasui, H., Ojima, T., 2019. Enzymatic production of xylooligosaccharides from red alga dulse (Palmaria sp.) wasted in Japan. Process Biochem. 82, 117-122. https://doi.org/10.1016/j.procbio.2019.03.030

**Claims**

1. Process for obtaining xylo-oligosaccharides from an algae, comprising the following steps:

   pre-treating the algae to obtain algae flakes with a size ranging from 1-10 mm; preferably 1.5 - 4 mm;
   fractioning the algae flakes by aqueous extraction, to obtain a soluble fraction and an insoluble fraction, wherein the soluble fraction comprises a soluble mixed-link $\beta1,3/\beta1,4$ xylan;
   hydrolysing the soluble fraction with 0.002 mg/L to 1 mg/L of endoxylanase, preferably at 15 to 30 °C, to obtain xylo-oligosaccharide, preferably xylo-oligosaccharides comprising mixed-link $\beta1,3/\beta1,4$ and homo-linked $\beta1,4$ xylo-oligosaccharides with a degree of polymerisation of at least 2-3;
   wherein the algae flakes comprise, on a dry weight basis:

   30-50% (w/w) of xylan,
   15-25% (w/w) of protein,
   20-30% (w/w) ash,
   2-10% (w/w) of lipids,
   1-10% (w/w) of glucose,
   3-15% (w/w) of galactose,
   0.1-0.5% (w/w) of phenolics.

2. Process according to the previous claim wherein the algae flakes comprise, on a dry weight basis:

   35-45% (w/w) of xylan;
   20-25% (w/w) of protein,
   20-25% (w/w) ash;
   3-8% (w/w) of lipids;
   1-5% (w/w) of glucose;
   3-8% (w/w) of galactose; and
   0.2-0.3% (w/w) of phenolics.

3. Process according to any of the previous claims wherein the concentration of endoxylanase ranges from 0.002 mg/L to 0.1 mg/L; preferably 0.002 mg/L to 0.01 mg/L.

4. Process according to any of the previous claims wherein the fractioning and hydrolysing steps are performed simultaneously.

5. Process according to any of the previous claims, wherein the algae class is selected from a group comprising *Cyanidiales, Porphyridialies, Bangiales, Acrochaetiales, Betrachospermales, Nemaliales, Corallinales, Palmariales, Gelidiales, Gracilariales,* or *Ceramiales*; preferably the algae species is selected from a group comprising *Palmaria palmata, Palmaria georfica, Palmaria decipiens, Palmaria marginicrassa, Palmaria hecatensis, Palmaria mollis, Palmaria stenogona, Palmaria moniliformis,* or *Palmaria callophylloides;* more preferably the algae species is *Palmaria palmata.*

6. Process according to any of the previous claims, wherein the aqueous extraction is carried out with 10-200 g/L of algae flakes suspended in distilled water, freshwater, sea water or salt water, for 1 to 12 hours, at a temperature ranging from 15 to 100 °C; preferably the aqueous extraction is carried out with agitation up to 120 rpm for 6 hours, at 15 - 25 °C.

7. Process according to any of the previous claims wherein at least 50% (w/w) of the total algae's xylan is extracted to the soluble fraction, preferably 60 to 80% (w/w).

8. Process according to any of the previous claims, wherein the soluble fraction comprises, on a dry weight basis:

> 50-70% (w/w) xylan, preferably 60-70% (w/w) xylan;
> up to 15% (w/w) galactose, preferably up to 10% (w/w) galactose;
> 1-5% (w/w) glucose, 20-25% (w/w) ash, 3-6% (w/w) protein and 0.4-0.8% (w/w) phenolics.

9. Process according to any of the previous claims further comprising a step of ultrafiltration and/or dialysis of the soluble fraction to remove low molecular weight compounds.

10. Process according to any of the previous claims, wherein the insoluble fraction comprises, on a dry weight basis:

> 45-55% (w/w) of protein;
> 10-20% (w/w) of xylan;
> 20-30% (w/w) ash;
> 0.1-0.5 % (w/w) of phenolics; and
> 7-10% (w/w) of lipids;

wherein the protein is 70-90% (w/w) of the total protein found in the processed algae.

11. Process according to any one of the previous claims, wherein the endoxylanase is a glycoside hydrolase family 8 or 11 endoxylanases (CAZy classification), preferably a glycoside hydrolase family 8 endoxylanase from *Pseudoalteromonas haloplanktis* TAH3a.

12. Soluble xylan composition obtainable by the process as described in any of the previous claims comprising mixed-link β1,3/β1,4 xylan.

13. Xylo-oligosaccharide composition obtainable by the process as described in any of the claims 1-12 comprising mixed-link β1,3/β1,4 and homo-linked β1,4 xylo-oligosaccharides with a degree of polymerisation of at least 2-3, wherein said composition comprises, on a dry weight basis, 50-70% (w/w) of xylo-oligosaccharides, preferably 60-70% (w/w); up to 15% (w/w) galactose; 1-5% (w/w) glucose; 20-25% (w/w) ash; 3-6% (w/w) protein; and 0.4-0.8% (w/w) phenolics.

14. Use of a soluble xylan composition as described in claim 12 as food/feed products, health, materials preparation, platform chemicals, biofuels, cosmetics, research and development and/or as substrate for enzyme activity measurements and screening.

15. Use of a xylo-oligosaccharide composition as described in claim 13 as a prebiotic; as an anti-cancer agent; as an antioxidant; enhancer of organoleptic properties in foods, feeds, or beverages; in health care; as an immunostimulatory agent; anti-inflammatory agent; anti-microbial agent; as an enzyme substrate; as xylo-oligosaccharide standards in quantitative assays; as reagent in the production of biofuels, platform chemicals and bioplastics; or as cosmetic ingredient.

Fig. 1

Fig. 2

**Fig. 3**

**Fig. 4**

Fig. 5

Europäisches
Patentamt
European
Patent Office
Office européen
des brevets

# EUROPEAN SEARCH REPORT

Application Number

**EP 21 19 3566**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KOBAYASHI MANAMI ET AL: "Identification of a Key Enzyme for the Hydrolysis of [beta]-(1->3)-Xylosyl Linkage in Red Alga Dulse Xylooligosaccharide from Bifidobacterium Adolescentis", MARINE DRUGS, vol. 18, no. 3, 1 March 2020 (2020-03-01), page 174, XP055879235, Basel, CH ISSN: 1660-3397, DOI: 10.3390/md18030174 * abstract; paragraph 4.2; fig.6 * | 1-15 | INV. C08B37/00 C08L5/00 |
| A,D | YAMAMOTO YOHEI ET AL: "Enzymatic production of xylooligosaccharides from red alga dulse (Palmariasp.) wasted in Japan", PROCESS BIOCHEMISTRY, vol. 82, 2019, pages 117-122, XP085696858, ISSN: 1359-5113, DOI: 10.1016/J.PROCBIO.2019.03.030 * paragraph 3.4 * | 1-15 | |

TECHNICAL FIELDS SEARCHED (IPC)

C08B
C08L

The present search report has been drawn up for all claims

| Place of search | Date of completion of the search | Examiner |
|---|---|---|
| Munich | 17 January 2022 | Pellegrini, Paolo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 960 772 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006002495 A
- EP 0265970 B1
- CN 101531722 B
- EP 3252083 A
- CN 108251472 A
- CN 108251472
- EP 0265970 A
- WO 2009117790 A
- JP 2001086999 B
- WO 2009117790 A2
- WO 2006002495 A1
- CN 101531722 A
- EP 3252083 A1

### Non-patent literature cited in the description

- **AACHARY, A.A. ; PRAPULLA, S.G.** Xylooligosaccharides (XOS) as an Emerging Prebiotic: Microbial Synthesis, Utilization, Structural Characterization, Bioactive Properties, and Applications. *Compr. Rev. Food Sci. Food Saf.,* 2011, vol. 10, 2-16, https://doi.org/10.1111/j.1541-4337.2010.00135
- **AHN, J.Y. ; KIL, D.Y. ; KONG, C. ; KIM, B.G.** Comparison of oven-drying methods for determination of moisture content in feed ingredients. *Asian-Australasian J. Anim. Sci.,* 2014, vol. 27, 1615-1622, https://doi.org/10.5713/ajas.2014.14305
- **AMORIM, C. ; SILVÉRIO, S.C. ; PRATHER, K.L.J. ; RODRIGUES, L.R.** From lignocellulosic residues to market: Production and commercial potential of xylooligosaccharides. *Biotechnol. Adv.,* 2019, vol. 37, 107397, https://doi.org/10.1016/j.biotechadv.2019.05.003
- **ÁVILA, P.F. ; FRANCO CAIRO, J.P.L. ; DAMASIO, A. ; FORTE, M.B.S. ; GOLDBECK, R.** Xylooligosaccharides production from a sugarcane biomass mixture: Effects of commercial enzyme combinations on bagasse/straw hydrolysis pretreated using different strategies. *Food Res. Int.,* 2020, vol. 128, 108702, https://doi.org/10.1016/j.foodres.2019.108702
- **BIELY, P. ; MISLOVICOVA, D. ; TOMAN, R.** Soluble chromogenic substrates for the assay of endo-1,4-beta-xylanases and endo-1,4-beta-glucanases. *Anal. Biochem.,* 1985, vol. 144, 142-146
- **BLIGH, E.G. ; DYER, W.J.** A rapid method of total lipid extraction and purifcation. *Can. J. Biochem. Physiol.,* 1959, vol. 37, 911-917, https://doi.org/10.1139/o59-099
- **COLLINS, T. ; GERDAY, C. ; FELLER, G.** Xylanases, xylanase families and extremophilic xylanases. *FEMS Microbiol. Rev.,* 2005, vol. 29, https://doi.org/10.1016/j.femsre.2004.06.005
- **COLLINS, T. ; MEUWIS, M.A. ; STALS, I. ; CLAEYSSENS, M. ; FELLER, G. ; GERDAY, C.** A novel family 8 xylanase, functional and physicochemical characterization. *J. Biol. Chem.,* 2002, vol. 277, 35133-35139, https://www.jbc.org/action/showPdf?pii=S0021-9258%2818%2936742-5
- **COURTIN, C.M. ; SWENNEN, K. ; VERJANS, P. ; DELCOUR, J.A.** Heat and pH stability of prebiotic arabinoxylooligosaccharides, xylooligosaccharides and fructooligosaccharides. *Food Chem.,* 2009, vol. 112, 831-837, https://doi.org/10.1016/j.foodchem.2008.06.039
- **DENIAUD, E. ; FLEURENCE, J. ; LAHAYE, M.** Interactions of the mix-linked beta-(1,3)/-(1,4)-d-xylans in the cell walls of Palmaria palmata (Rhodophyta). *J. Phycol.,* 2003, vol. 39, 74-82, https://doi.org/10.1046/j.1529-8817.2003.02047.x
- **HSU, C.K. ; LIAO, J.W. ; CHUNG, Y.C. ; HSIEH, C.P. ; CHAN, Y.C.** Xylooligosaccharides and fructooligosaccharides affect the intestinal microbiota and precancerous colonic lesion development in rats. *J Nutr,* 2004, vol. 134, 1523-1528, https://doi.org/10.1093/jn/134.6.1523
- **KABEL, M.A. ; KORTENOEVEN, L. ; SCHOLS, H.A. ; VORAGEN, A.G.J.** In vitro fermentability of differently substituted xylo-oligosaccharides. *J. Agric. Food Chem.,* 2002, vol. 50, 6205-6210, https://doi.org/10.1021/jf020220r
- **KIM, M.-J. ; YOO, S.-H. ; JUNG, S. ; PARK, M.-K. ; HONG, J.-H.** Relative Sweetness, Sweetness Quality, and Temporal Profile of Xylooligosaccharides and Luo Han Guo (Siraitia grosvenorii) Extract. *Food Sci. Biotechnol,* 2015, vol. 24, 965-973, https://doi.org/10.1007/s10068-015-0124-x

- **LAURENS, L.M.L. ; DEMPSTER, T.A. ; JONES, H.D.T. ; WOLFRUM, E.J. ; VAN WYCHEN, S. ; MCALLISTER, J.S.P. ; RENCENBERGER, M. ; PARCHERT, K.J. ; GLOE, L.M.** Algal biomass constituent analysis: method uncertainties and investigation of the underlying measuring chemistries. *Anal. Chem.,* 2012, vol. 84, 1879-1887, https://doi.org/10.1021/ac202668c
- **MACHU, L. ; MISURCOVA, L. ; VAVRA AMBRO-ZOVA, J. ; ORSAVOVA, J. ; MLCEK, J. ; SOCHOR, J. ; JURIKOVA, T.** Phenolic content and antioxidant capacity in algal food products. *Molecules,* 2015, vol. 20, 1118-1133, https://doi.org/10.3390/molecules20011118
- **MAEDA, R. ; IDA, T. ; IHARA, H. ; SAKAMOTO, T.** Induction of apoptosis in MCF-7 cells by $\beta$-1,3-xylooligosaccharides prepared from Caulerpa lentillifera. *Biosci. Biotechnol. Biochem.,* 2012, https://doi.org/10.1271/bbb.120016
- **MCCLEARY, B. V. ; MCGEOUGH, P.** A comparison of polysaccharide substrates and reducing sugar methods for the measurement of endo-1,4-$\beta$-xylanase. *Appl. Biochem. Biotechnol.,* 2015, vol. 177, 1152-1163, https://doi.org/10.1007/s12010-015-1803-z
- **TELEMAN, A. ; LUNDQVIST, J. ; TJERNELD, F. ; STÅLBRAND, H. ; DAHLMAN, O.** Characterization of acetylated 4-O-methylglucuronoxylan isolated from aspen employing 1H and 13C NMR spectroscopy. *Carbohydr. Res.,* 2000, vol. 329, 807-815, https://doi.org/10.1016/S0008-6215(00)00249-4
- **WANG, Y. ; ZHANG, J.** A novel hybrid process, enhanced by ultrasonication, for xylan extraction from corncobs and hydrolysis of xylan to xylose by xylanase. *J. Food Eng.,* 2006, vol. 77, 140-145, https://doi.org/10.1016/j.jfoodeng.2005.06.056
- The Lowry method for protein quantitation. **WATER-BORG, J.H. ; MATTHEWS, H.R.** Basic Protein and Peptide Protocols. Humana Press, 2003, 1-4
- **YAMAMOTO, Y. ; KISHIMURA, H. ; KINOSHITA, Y. ; SABURI, W. ; KUMAGAI, Y. ; YASUI, H. ; OJI-MA, T.** Enzymatic production of xylooligosaccharides from red alga dulse (Palmaria sp.) wasted in Japan. *Process Biochem.,* 2019, vol. 82, 117-122, https://doi.org/10.1016/j.procbio.2019.03.030